# EUROPEAN PATENT APPLICATION

(11) **EP 1 424 649 A2**
(43) Date of publication of application: **02.06.2004**
(21) Application number: 03026660.5
(22) Date of filing: 19.11.2003
(51) Int. Cl.: G06F 19/00

(54) **Medical service assisting system, medical service assisting method, and program thereof**

(30) Priority: 28.11.2002 JP 2002345814
(71) Applicant: Olympus Corporation, Tokyo 151-0072 (JP)
(72) Inventor: Ohishi, Hiroko, Hachioji-shi Tokyo 193-0832 (JP)
(74) Representative: von Hellfeld, Axel, Dr. Dipl.-Phys.

(57) **Abstract**

A medical service assisting system assisting in a service rendered in a medical institution comprises:
a preprocedure information inputting unit (1) inputting preprocedure information which is composed of at least one of a type of a drug used for a preprocedure performed before an examination is conducted, an amount of the drug, an appliance used for the preprocedure, and the name of a person who performs the preprocedure; a condition inputting unit (1) inputting patient condition information, which is information indicating the condition of a patient after the preprocedure; and a storing unit (4) correlating and storing the preprocedure information, the patient condition information, and a time of the preprocedure.

## Description

### Cross Reference to Related Application

This application is based on and claims the benefit of priority from the prior Japanese Patent Application No. 2002-345814 filed in Japan on Nov. 28, 2002, the entire contents of which are incorporated by this reference.

### Background of the Invention

The present invention relates to a system, a method, and a program for making the effective use of patient information on a medical scene.

### Description of the Related Art

In recent years, an electronic endoscope where a solid-state image capturing element is used as an image capturing means at the tip has come into widespread use as a replacement for a fiber scope that observes a coelom by using an image guide formed with an optical fiber.

At this time, a shot image is stored in a storage device such as a hard disk (HDD) as electronic data. At the same time, information of a person being tested (patient) is also corresponded to the image, and stored. Those information items are used in various situations of medical diagnosis and treatment within a hospital, and contribute to an increase in the efficiency of medical services.

As a system intended to manage such data of endoscopy, Japanese Patent Publication No. 2002-73615 discloses a medical filing system that can handle necessary information at right timing in a service flow for making an examination.

With this system, examination request information, etc. are firstly input, and a drug, etc. used for a preprocedure (a procedure preparatory to an examination prior to the examination), and contents of the preprocedure (such as anesthesia medication, injection, purgative medication, etc.) are input. Thereafter, information accompanying the conduct of the examination is input, and an image shot with an endoscope is correlated with these pieces of data, and stored.

### Summary of the Invention

A medical service assisting system assisting in a service rendered in a medical institution according to the present invention comprises:
a preprocedure information inputting unit inputting preprocedure information which is composed of at least one of a type of a drug used for a preprocedure performed before an examination is conducted, an amount of the drug, an appliance used for the preprocedure, and a name of a person who performs the preprocedure;
a condition inputting unit inputting patient condition information, which is information indicating the condition of a patient after the preprocedure; and
a storing unit correlating and storing the preprocedure information, the patient condition information, and a time of the preprocedure.

Preferably, the above described medical service assisting system further comprises
a correlated information presenting unit presenting the correlation among the preprocedure information, the patient condition information, and the time of the preprocedure, which are stored in the storing unit.

Preferably, the above described medical service assisting system further comprises:
an examination reservation displaying unit displaying examination reservation information, which is reservation information of the examination; and
a display controlling unit controlling the display form of the examination reservation information displayed by the examination reservation displaying unit, based on the patient condition information.

### Brief Description of the Drawings

Fig. 1 schematically shows the configuration of an endoscopic information system according to the present invention;
Fig. 2 schematically shows the fundamental configuration of the main body of a PC;
Fig. 3 shows a screen displayed when the endoscopic information system is started up;
Fig. 4 shows a "preprocedure input" screen;
Fig. 5 shows the flow of a proprocedure input process;
Fig. 6 shows a "performance contents entry" screen;
Fig. 7 shows the flow of a process for making verification before an examination is started;
Fig. 8 shows a "patient acceptance list" screen;
Fig. 9 shows the flow of an examination reservation process; and
Fig. 10 shows the flow of a process for displaying patient information in the "patient acceptance list".

### Description of the Preferred Embodiments

A preferred embodiment according to the present invention is described below.

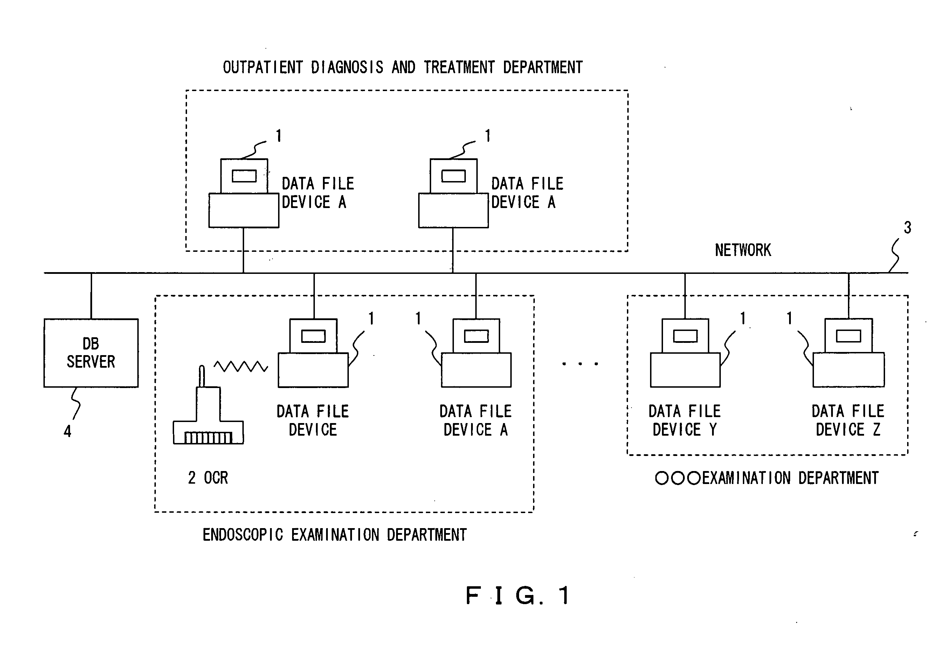

Fig. 1 schematically shows the configuration of an endoscopic information system according to the present invention. As shown in Fig. 1, various departments such as an outpatient diagnosis and treatment department, an endoscopic examination department, etc. exist on this system, and each of the departments has one or a plurality of personal computers and data file devices (hereinafter referred to as PCs) 1. These PCs 1 are connected by a network such as a LAN (Local Area network) 3, etc., and comprise an input device such as an OCR 2, a keyboard, a mouse, etc.

Additionally, a database server 4 exists on the network, and stores patient information and image data, which are input from each of the PCs 1. The endoscopic examination department is described in this preferred embodiment.

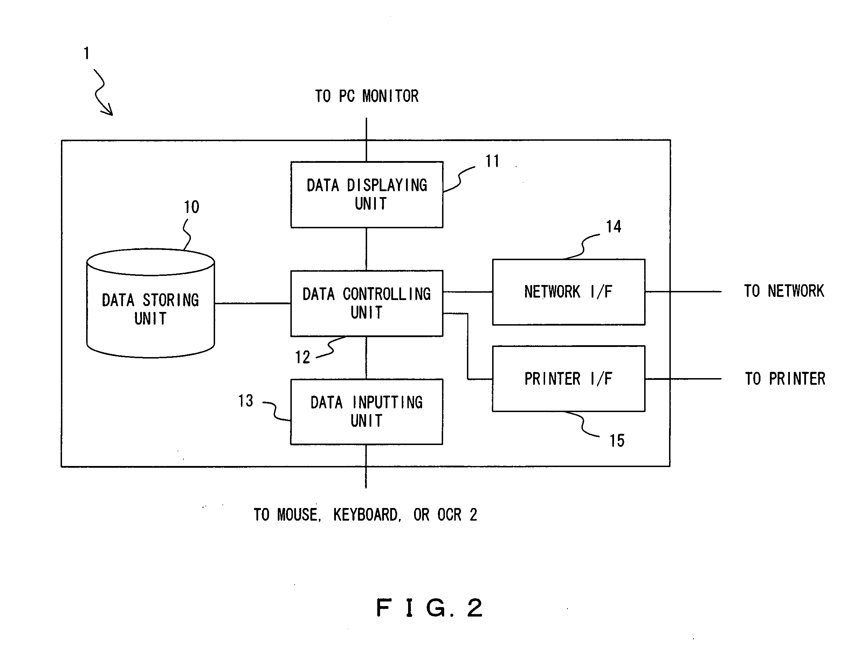

Fig. 2 schematically shows the fundamental configuration of the main body of each of the PCs 1.
The main body of each of the PCs 1 is configured by: a data controlling unit 12 performing a process or an arithmetic operation in accordance with a program; a data storing unit 10 storing various pieces of data such as image data, patient information, etc.; a data displaying unit 11 making a PC monitor display image data, etc.; a data inputting unit 13 intended to input a command, data, etc. from an input device such as a mouse, a keyboard, a portable reading device typified by an OCR 2, etc.; a network I/F (interface) 14, which is an interface for connecting with a network such as a LAN 3, etc.; and a printer I/F (interface) 15, which is an interface for outputting data to a printer.

At this time, the portable reading device, which is one of input devices and typified by the OCR 2, may be connected to the main body of the PC 1 wiredly or wirelessly. Also a PDA (Personal Digital Assistant) may be available as the portable reading device. Or, an RFID (Radio Frequency IDentification) tag typified by a transponder (a medium that can read and write information wirelessly), an RFID reader, etc. may be available. As described above, a mechanically readable identifier, a mechanically readable means, or an optically reading means is used as the reading device.

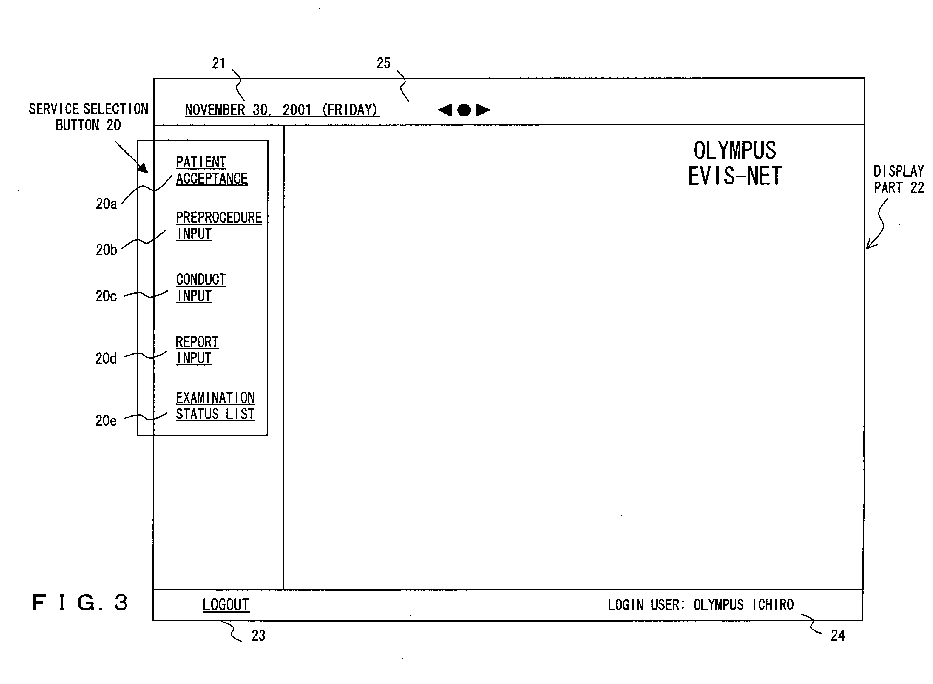

Contents of the system are described below. Fig. 3 shows a screen displayed when this system running on a PC 1 is started up. This screen is configured by: service selection buttons 20 composed of a "patient acceptance" button 20a, a "preprocedure input" button 20b, a "conduct input" button 20c, a "report input" button 20d, and an "examination status list" button 20e; a date display part 21; a display part 22; a "logout" button 23; a login user name display part 24; and a date change part 25. With the click of each of the service selection buttons 20, each service screen is displayed. Since Fig. 3 assumes the time point when this system is started up, a start screen is displayed in the display part 22.

Additionally, the date of a current day is displayed in the date display part 21, and the date can be changed by operating the date change part 25. With the click of the "logout" button 23, the system can be terminated. In the login user name display part 24, the name of a user who currently logs in to the system is displayed.

The "patient acceptance" button 20a, the "preprocedure input" button 20b, and the "conduct input" button 20c among the service selection buttons 20 will be described later. The "report input" button 20d is intended to input diagnostic findings, etc., and the "examination status list" button 20e is intended to display an examination status list. However, since these buttons do not relate to the present invention, they are not referred to further.

Description is provided in accordance with the flow of an actual diagnosis and treatment service. This preferred embodiment is described by taking as an example a patient whose name is Olympus Taro (hereinafter referred to as a patient A), and who undergoes a preprocedure.

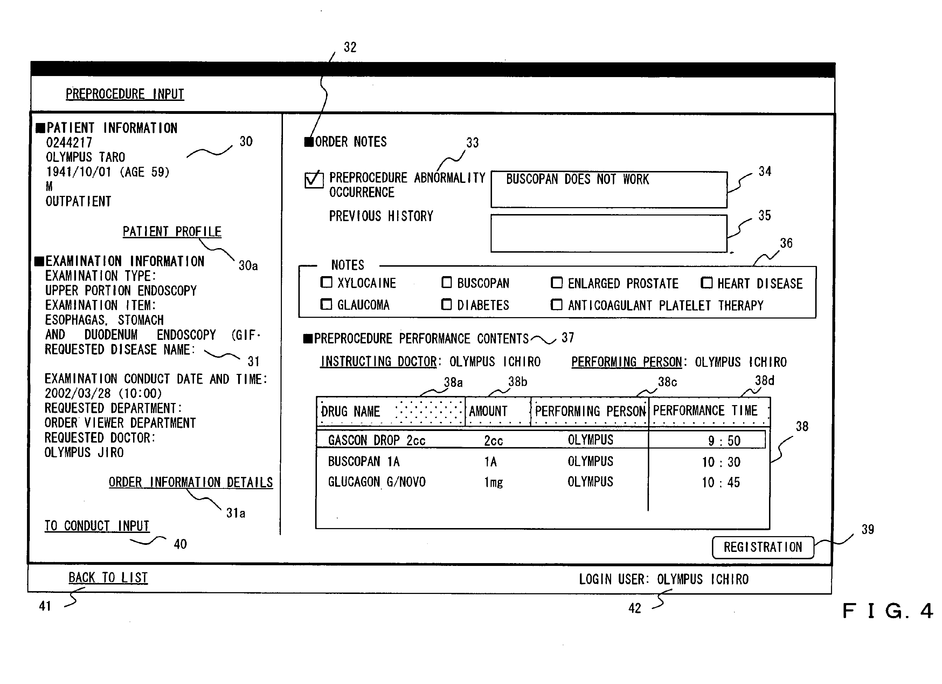

Firstly, an operator of the system clicks the "preprocedure input" button 20b to display a "preprocedure input list" screen (not shown). When the operator selects the patient A from the list, a "preprocedure input" screen shown in Fig. 4 is displayed. This "preprocedure input" screen is configured by: a "patient information" display field 30; an "examination information" display field 31; an "order notes" field 32 (a "preprocedure abnormality occurrence" checkbox 33, an "abnormality information description" text box 34, a "previous history" text box 35, and a "notes" field 36); a "preprocedure performance contents" field 37; a "preprocedure performance contents list" 38; a "registration" button 39; a "to-conduct-input" button 40; a "back-to-list" button 41; and a "login user name display part" 42.

In the "patient information" display field 30, a "patient ID", "name", "date of birth", "sex", etc. are displayed. In this "patient information" display field, 30, a "patient profile" button 30a further exists. With the click of the "patient profile" button 30a, a screen on which information items such as a "blood type", "height/weight", "allergies", "disabilities", "infectious diseases", "diseases", "notes", "sample test result", "previous medication information", etc. can be referenced is displayed.

In the "examination information" display field 31, information items such as an "examination type", an "examination item", a "requested disease name", "examination scheduled date and time", a "requested department", a "requested doctor", etc. are displayed. In this "examination information" display field 31, an "order information details" button 31a further exists. With the click of the "order information details" button 31a, detailed information such as a requesting department, etc. are displayed.

Information displayed in the "patient information" display field 30 and the "examination information" display field 31 are information provided from an HIS (Hospital Information System), which is a higher-order system, to a department system.

The "order notes" field 32 is described. If the "preprocedure abnormality occurrence" checkbox 33 is checked to be set to ON, this enables an input to the "abnormality information description" text box 34. Or, if the "preprocedure abnormality occurrence" checkbox 32 is set to OFF, this disables an input to the "abnormality information description" text box 34. Additionally, contents input in the past up to the preceding time to the "abnormality information description" text box 34 or an "abnormality information description" text box 58 to be described later, and an "examination cancellation reason" text box 61 are displayed in the "previous history" display part 35.

In the "notes" field 36, a checkbox of a corresponding item is set to ON among items such as "xylocaine shock", "buscopan shock", "enlarged prostate", "heart disease", "glaucoma", "diabetes", and "anticoagulant platelet therapy".

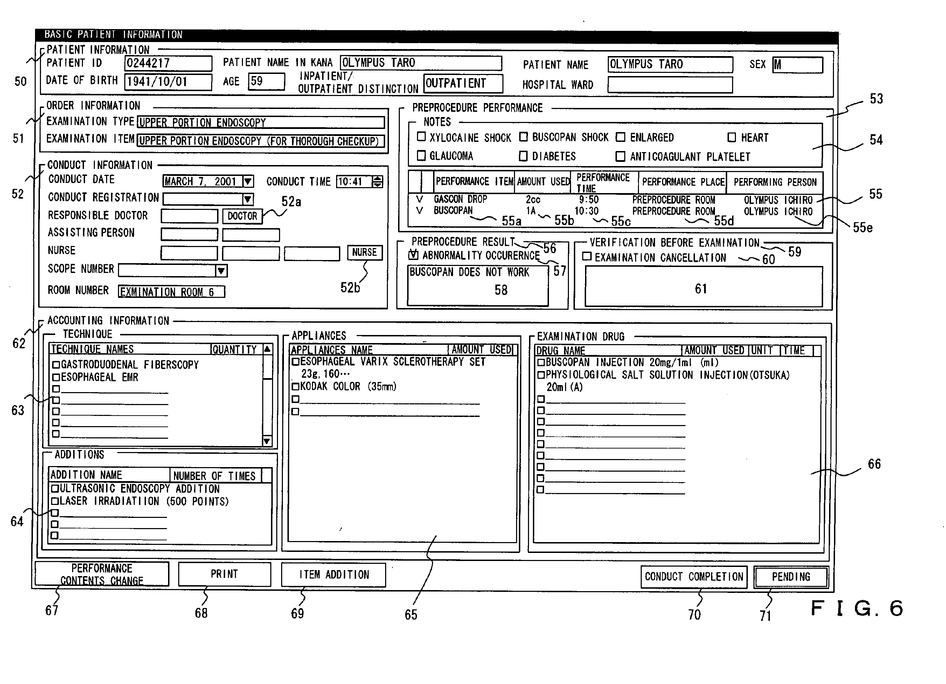

In the "preprocedure performance contents" field 37, an "instructing doctor", a "performing person", a "preprocedure performance contents list" 38 are displayed. As the "instructing doctor", the name of a doctor who instructs a preprocedure is displayed. As the "performing person", the name of a person who performs the preprocedure is displayed. The "preprocedure performance contents list" 38 is composed of items such as a "drug name" 38a, an "amount" 38b, a "performing person" 38c, and a "performance time" 38d. Additionally, with the click of the "to-conduct-input" button 40, a "performance contents entry" screen, which is shown in Fig. 6 and will be described later, is displayed. With the click of the "back-to-list" button 41, this screen is closed, and the display goes back to the "preprocedure input list" screen, which is the transition source. The login user name display part 42 is similar to the login user name display part 24 shown in Fig. 3.

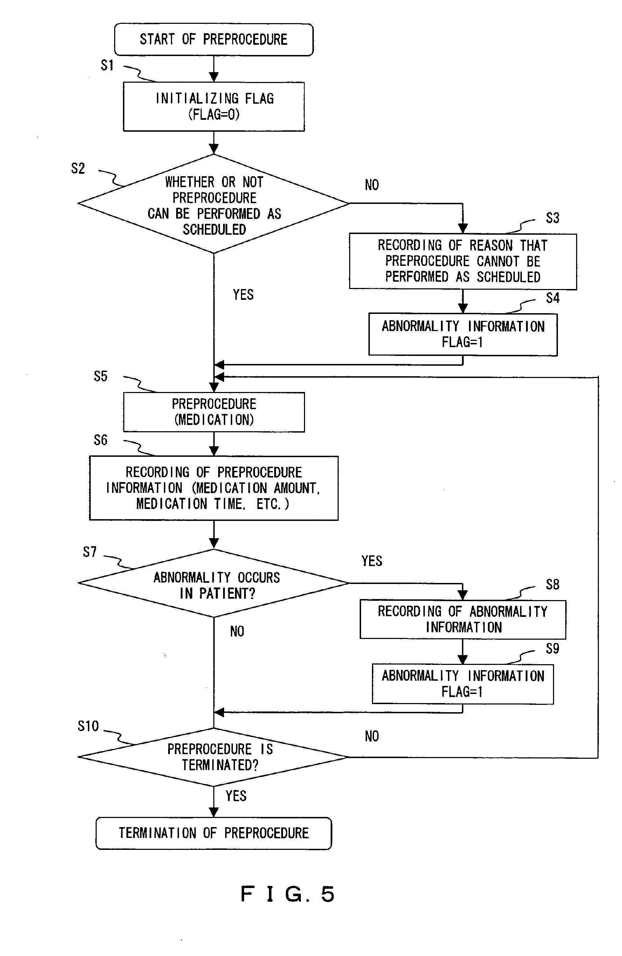

The preprocedure input process is described step by step with reference to the flow of Fig. 5.

When the "preprocedure input" screen is displayed, this system initializes an abnormality information flag to be reset to 0 (step S1. Hereinafter, step is abbreviated to S).

Next, an operator determines whether or not a preprocedure for the patient A can be performed as scheduled. Here, "whether or not a preprocedure for the patient A can be performed as scheduled" assumes, for example, that the preprocedure for the patient A cannot be performed within a scheduled timeframe due to some reason. If the preprocedure can be performed as scheduled in S2, the process proceeds to S5.

If the preprocedure cannot be performed as scheduled, the operator checks the "preprocedure abnormality occurrence" checkbox 33, and inputs this information to the "abnormality information description" text box 34 (S3). At this time, the abnormality information flag is set to 1 (S4), and the process proceeds to S5.

After the above described operations, the operator performs the preprocedure for the patient (S5), and records preprocedure information such as the name of a drug used, the amount of the drug, a medication time, etc. (S6). Here, S6 is specifically described. A label read by an OCR is affixed to a bottle of a medicated drug. At the time of medication, its OCR information is read with the OCR 2. For a single-use drug, even its use amount (quantity) can be read with the OCR 2. In the meantime, for a drug which is not single-use, its use amount (quantity) can be obtained by separately reading a list, in which only use amounts (quantities) are put into OCR information, with the OCR 2. Also a time required to read the amount with the OCR 2 is obtained at this time.

When the OCR information of the drug is read with the above described operation, the read information is displayed in the "preprocedure performance contents list" 38. In Fig. 4, "drug name: gascon drop", "amount: 2cc", "performing person: Olympus Ichiro", "performance time: 9:50" are displayed.

Following the medication in S5, the operator determines whether or not an abnormality occurs in the condition of the patient (S7). If an abnormality does not occur in the condition of the patient in S7, the process proceeds to S10. If an abnormality occurs in the condition of the patient in S7, the operator checks the "preprocedure abnormality occurrence" checkbox 33, and inputs this information to the "abnormality information description" text box 34 (S8). At this time, the abnormality information flag is set to 1 (S9), and the process proceeds to S10.

Following the above described operations, the operator determines whether or not the preprocedure is terminated (S10). If the preprocedure is not terminated yet, the operations in S5 to S10 are repeated. When the entire preprocedure is completed, the preprocedure is determined to be terminated.

In Fig. 4, three drugs are used for the preprocedure, and a description that "the drug named buscopan (which is administered at 10:30) does not work" is provided in the "abnormality information description" text box 34.

Upon termination of the preprocedure, the operator clicks the "registration" button 39, so that the contents input to the "preprocedure abnormality occurrence" checkbox 33, the "abnormality information description" text box 34, the "notes" 36, the "preprocedure performance contents list" 38 (including also the "instructing doctor" information, and the "performing person" information) are correlated and registered to the database server 4.

Also for an appliance used for the preprocedure, or the like, an item such as an "appliance used" may be newly added to this screen so that an appliance used can be input on the screen of Fig. 4. In this case, with the click of the above described "registration" button 39, an appliance name input to the "appliance used" item is registered to the database server 4 similar to the other items.

Verification made before an examination is started is described next. After the preprocedure is terminated, the patient A is guided to an examination room, and undergoes an examination. Prior to the examination, it is verified whether or not the patient A is in a state where he or she can undergo the examination. The verification made before the examination is started is described step by step.

Firstly, the operator clicks the "conduct input" button 20c shown in Fig. 3 in the examination room. After the click, the "performance contents entry list" screen is displayed, When information of the patient A is selected from the list, the "performance contents entry" screen shown in Fig. 6 is displayed.

The "performance contents entry" screen is configured by a "basic patient information" field 50, an "order information" field 51, a "performance information" field 52, a "preprocedure performance item" field 53, and an "accounting information" field 62.

In the "basic patient information" field 50, a "patient ID", a "patient name in kana", a "patient name", "sex", "date of birth", "age", inpatient/outpatient distinction (distinction between an inpatient and an outpatient), and a "hospital ward" (displayed only for an inpatient) are displayed. In the "order information" field 51, an "examination type", and an "examination item" are displayed. The "basic patient information" field 50 and the "order information" field 51 are information provided from the HIS.

To the "performance information" field 52, "performance date", "performance time", a "performance registrant", a "responsible doctor", an "assisting doctor", a "nurse", a "scope number", and a "room number" are input. For the "responsible doctor", a screen on which a responsible doctor is selected is displayed with the click of a responsible doctor selection button 52a, and a responsible doctor can be also selected on that screen. Additionally, for the "nurse", a screen on which a nurse is selected is displayed with the click of a nurse selection button 52b, and a nurse can be also selected on that screen.

In the "accounting information" field 62, a "technique list" 63, an "addition list" 64, an "appliance list" 65, and an "examination drug list" 66 are displayed. Corresponding items within the lists, which are used for an examination, are checked to be set to ON. Furthermore, at the bottom of the screen, a "performance contents change" button 67, a "print" button 68, an "item addition" button 69, a "conduct completion" button 70, and a "pending" button 71 are provided.

The preprocedure performance item 53 is configured by: a "notes" field 54 where a plurality of notes such as "xylocaine shock", "buscopan shock", "enlarged prostate", "heart disease", "glaucoma", "diabetes", and "anticoagulant platelet therapy" are represented as checkboxes; a "preprocedure performance contents list" 55 (composed of a "performance item" 55a, a "performance amount" 55b, a "performance time" 55c, a "performance place" 55d, a "performing person" 55e, etc.); a "preprocedure result" field 56 composed of an "abnormality occurrence" checkbox 57, and an "abnormality information description" text box 58; and a "verification-before-examination" field 59 composed of an "examination cancellation" checkbox 60, and an "examination cancellation reason" text box 61.

The "preprocedure performance item" 53 is based on the information input on the "preprocedure input" screen described with reference to Fig. 4, and the information registered to the database server 4 on that screen is displayed in this item. The "notes" field 54 corresponds to the "notes" field 36 on the "preprocedure input" screen. The "preprocedure performance contents list" 55 corresponds to the "preprocedure performance contents list" 38. The "abnormality occurrence" checkbox 57 corresponds to the "preprocedure abnormality occurrence" checkbox 33. The "abnormality information description" text box 58 corresponds to the "abnormality information description" text box 35. With the "preprocedure performance contents list" 55, the name and the amount of a drug, the time point when the drug is administered, the place where the drug is administered, and the name of a person who administers the drug can be found at a glance.

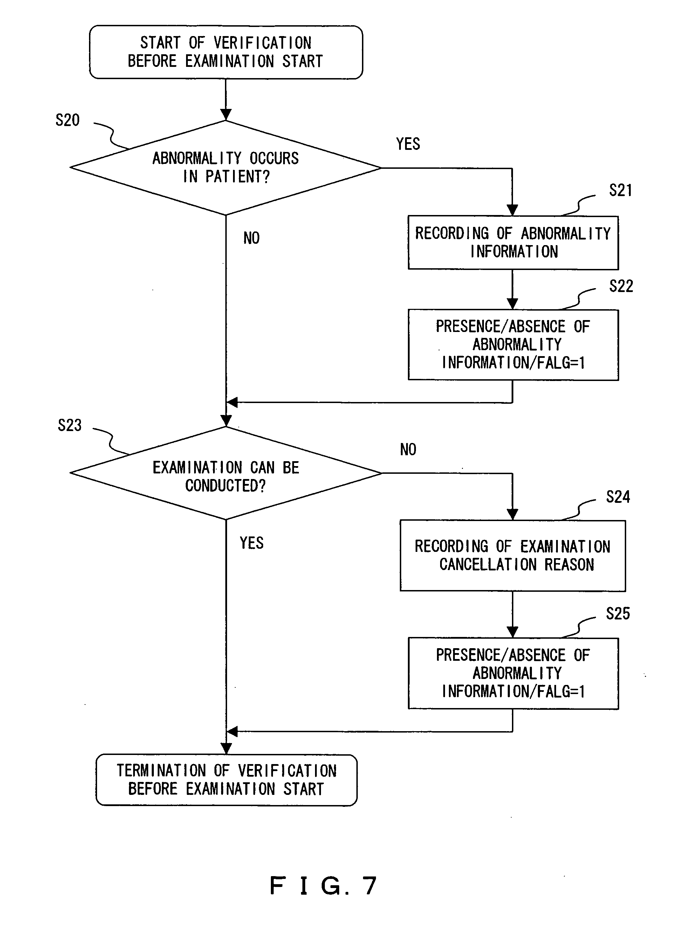

The process for making verification before an examination is started is described step by step with reference to Fig. 7. The operator determines whether or not an abnormality occurs in the condition of the patient A (S20). If an abnormality is determined not to occur in the condition of the patient as a result of the determination made in S20, the process proceeds to S23. If an abnormality is determined to occur in the condition of the patient as a result of the determination made in S20, the operator checks the "abnormality occurrence" checkbox 57, and inputs this information to the "abnormality information description" text box 58 (S21). At this time, the abnormality information flag is set to 1 (S22), and the process proceeds to S23.

Next, the operator determines whether or not an examination can be conducted for the patient (S23). If it is determined in S23 that the examination can be conducted for the patient, this flow is terminated. Or, if it is determined in S23 that the examination cannot be conducted for the patient, the operator checks the "examination cancellation" checkbox 60, and inputs this information to the "examination cancellation reason" text box 61 (S24). At this time, the abnormality information flag is set to 1 (S25), and the verification made before the examination is started is terminated.

After the above described verification done before the examination is made, the examination is conducted or canceled, and information corresponding to the contents of the examination is input. Then, with the click of a "conduct completion" button 70 or a "pending" button 71, this information is correlated with the preprocedure performance item 53, and registered to the database server 4.

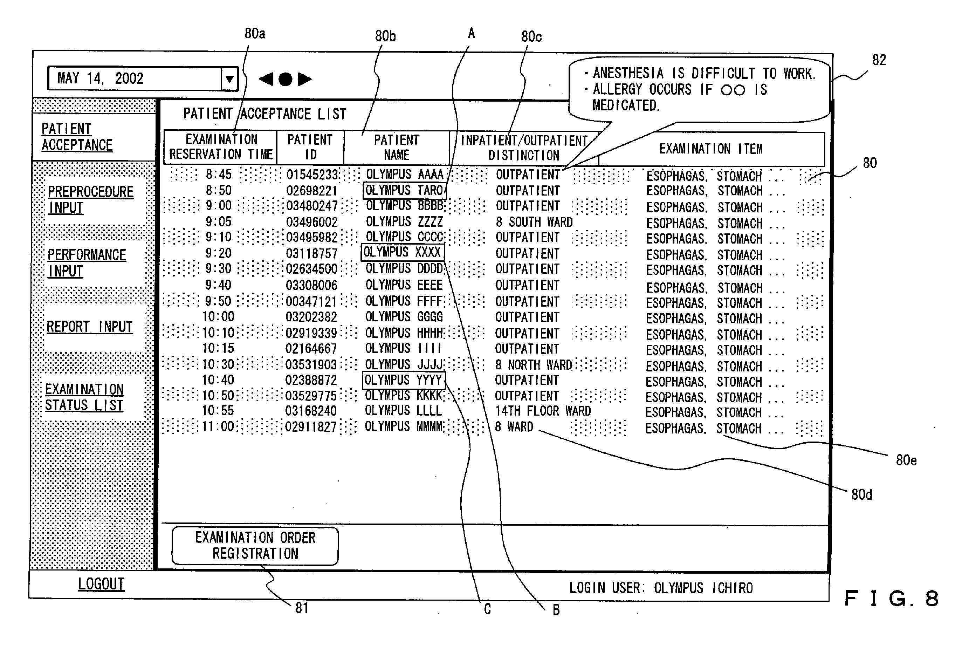

Then, a reservation of the next examination of the patient A is made. The operator clicks the "patient acceptance" button 20a shown in Fig. 3, so that a "patient acceptance list" screen shown in Fig. 8 is displayed. The "patient acceptance list" screen is configured by: a patient acceptance list 80 composed of an "examination reservation time" 80a, a "patient ID" 80b, a "patient name" 80c, an "inpatient/outpatient distinction" 80d, and an "examination item" 80e; and an "examination order registration" button 81. Furthermore, a logout button and a login user name display part, which are positioned at the bottom of this screen, are similar to the logout button 23 and the login user name display part 24, which are shown in Fig. 3.

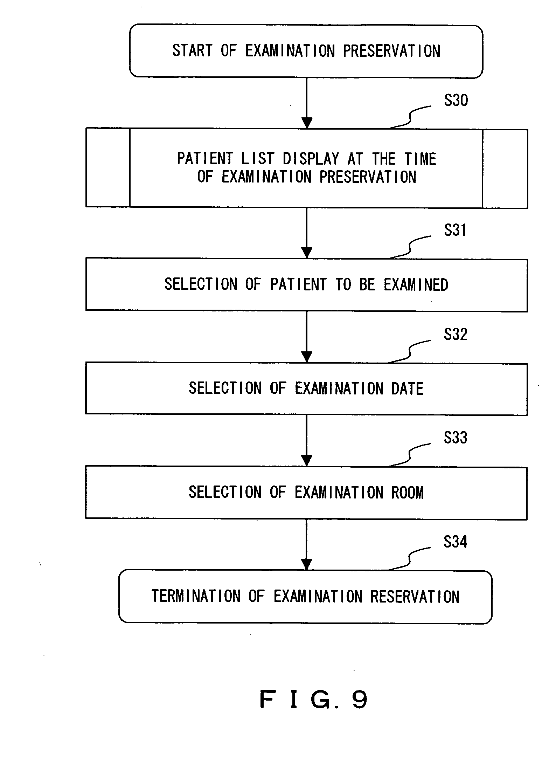

The service or the process flow on the "patient acceptance list" screen is in accordance with an examination reservation flow shown in Fig. 9. Firstly, when the "patient acceptance list" screen is displayed, patient information is displayed in the patient acceptance list (S30). Although this reservation information is provided from the HIS, reservation information can be also input on this screen. If reservation information is input on this screen, a patient for whom an examination reservation is to be made is selected from the patient acceptance list 80 (the patient A is selected in this preferred embodiment), and the "examination order registration" button 81 is clicked, so that a screen for reserving an examination is displayed. Then, the date of the next examination of the patient is selected on that screen (S32), an examination room is selected (S33), and the examination reservation is terminated.

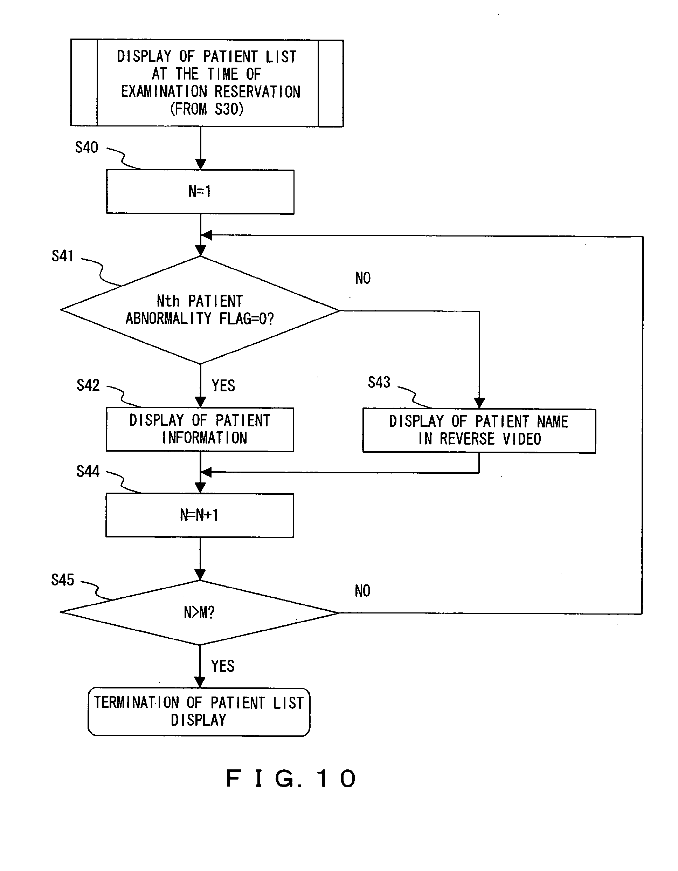

Here, S30 is described in detail with reference to Fig.10. The process flow shown in Fig. 10 is intended to display patient information with abnormality information in reverse video so as to make a distinction between patient information with abnormality information and that without abnormality information, when patient information is displayed in the patient acceptance list 80. The process flow of Fig. 10 is described below.

The process proceeds from S30 to S40. At this time, a PC 1 obtains reservation/acceptance information of the patient (patient information to be displayed on the "patient acceptance list" screen), which is provided from the HIS, further obtains abnormality information, etc. correlated with that information from the database server 4, and stores the obtained number of pieces of patient information as a variable M (M is a positive number). Then, a variable N (N is a positive number) is initialized to 1 (S40).

Next, it is determined whether or not an abnormality information flag possessed by the patient information, which is displayed as the N=1st of the patient acceptance list 80, is 0 (S41). If the abnormality information flag is 1, this patient information is displayed in reverse video in the "patient acceptance list" (S43), and the flow proceeds to S44. Or, if the abnormality information flag is 0, the patient information is not displayed in reverse video in the "patient acceptance list" (S42), and the flow proceeds to S44.

In S44, the variable N is incremented. If the variable N is equal to or smaller than the number of pieces of patient information to be displayed M in S45, the flow returns to S41. Then, the operations in S41 to S45 are repeated. If the variable N becomes larger than the number of pieces of patient information to be displayed M in S45, this flow is terminated, and the process proceeds to S31 of Fig. 9.

Patients A, B, C in Fig. 8 indicate patient information displayed in reverse video in accordance with the flow of Fig. 10. When a mouse cursor is positioned on any of the patient information displayed in reverse video at this time, abnormality information possessed by the patient information is displayed in a popup window 82 as shown in Fig. 8. This abnormality information is abnormality information that is input in the "preprocedure performance item" field 53 on the "preprocedure input" screen or the "performance contents entry" screen.

Furthermore, the above described abnormality information is effectively used at the time of the next preprocedure. For example, on the "preprocedure input" screen shown in Fig. 4, a history of abnormality information (abnormality information obtained from the database server 4) until the preceding preprocedure is displayed on the "previous history" display part 35. Therefore, a suitable preprocedure in consideration of that information can be performed. Accordingly, the recorded previous preprocedure information is fed back and displayed on a screen, etc. at the time of the next examination. This prevents a problem where anesthesia is difficult to work due to the performance of the same preprocedure as before, or the repetitive use of the same drug.

As described above, using the system according to the present invention facilitates the recording of preprocedure activities. Furthermore, attention is made not to cause problems similar those to that have occurred in the past, at the next or subsequent times, and the safety and the efficiency of a preprocedure can be improved.

Additionally, the name of a drug used for a preprocedure, the name of a person who uses the drug, and the time point when the drug is used, and the amount of the drug are accurately recorded, and can be referenced depending on need. Therefore, the contents of the preprocedure can be accurately traced.

Accordingly, as described above, using the present invention facilitates the recording of preprocedure activities. Additionally, its recorded information is fed back and displayed at the time of an examination conducted after the preprocedure, or a reservation of the next examination, whereby a problem similar to that at the time of the preprocedure can be prevented.

Additionally, information items such as the type and the amount of a drug used for a preprocedure, an appliance used, the name of a person who performs the preprocedure, and a preprocedure time are correlated and stored. Therefore, the name of a person who uses a drug for a preprocedure, the time point when the drug is used, the name and the amount of the drug, etc. can be accurately recorded. Besides, these information items can be referenced depending on need, whereby the contents of the preprocedure can be accurately traced.

Furthermore, abnormality information of particular patient information can be referenced, so that a reservation in consideration of that information can be made.

Still further, information required for a preprocedure can be input regardless of location at the time of the preprocedure. This can improve the real-time nature of a preprocedure information input, leading to improvements in data realiability.

Still further, information required for a preprocedure can be mechanically input with an OCR, thereby reducing time taken for an input. This can improve the real-time nature. Besides, manual input errors can be prevented from occurring, leading to improvements in data reliability.

Still further, predetermined information possessed by predetermined patient information is displayed in a popup window for the patient information on an examination reservation list screen. As a result, a reservation of the next examination in consideration of that information can be made in order to prevent a problem similar to that at the time of a preprocedure from occurring.

Still further, predetermined information of a previous preprocedure is displayed on the preprocedure input screen, whereby a problem similar to that at the time of the preprocedure can be prevented from occurring.

## Claims

1. A medical service assisting system assisting in a service rendered in a medical institution, comprising:
a preprocedure information inputting unit inputting preprocedure information which is composed of at least one of a type of a drug used for a preprocedure performed before an examination is conducted, an amount of the drug, an appliance used for the preprocedure, and a name of a person who performs the preprocedure;
a condition inputting unit inputting patient condition information, which is information indicating a condition of a patient after the preprocedure; and
a storing unit correlating and storing the preprocedure information, the patient condition information, and a time of the preprocedure.

2. The system according to claim 1, further comprising
a correlated information presenting unit presenting a correlation among the preprocedure information, the patient condition information, and the time of the preprocedure, which are stored in the storing unit.

3. The system according to claim 1, further comprising:
an examination reservation displaying unit displaying examination reservation information, which is reservation information of the examination; and
a display controlling unit controlling a display form of the examination reservation information displayed on the examination reservation displaying unit, based on the patient condition information.

4. The system according to claim 1, wherein
the preprocedure information inputting unit is a portable terminal.

5. The system according to claim 1, wherein
the preprocedure information inputting unit is a portable reading device.

6. The system according to claim 3, wherein
the display controlling unit displays predetermined information possessed by patient condition information corresponding to predetermined examination reservation information if only the predetermined examination reservation information is selected from among the examination reservation information displayed on the examination reservation displaying unit, based on the patient condition information.

7. The system according to claim 3, wherein
the display controlling unit performs marking for the examination reservation information displayed on the examination reservation displaying unit in color.

8. The system according to claim 1, further comprising
a history information displaying unit displaying a history of the patient condition information stored in the storing unit.

9. A medical service assisting method assisting in a service rendered in a medical institution, comprising:
inputting preprocedure information which is composed of at least one of a type of a drug used for a preprocedure performed before an examination is conducted, an amount of the drug, an appliance used for the preprocedure, and a name of a person who performs the preprocedure;
inputting patient condition information, which is information indicating a condition of a patient after the preprocedure; and
correlating and storing the preprocedure information, the patient condition information, and a time of the preprocedure.

10. The method according to claim 9, further comprising
presenting a correlation among the preprocedure information, the patient condition information, and the time of the preprocedure, which are stored.

11. The method according to claim 9, further comprising:
displaying examination reservation information, which is reservation information of the examination; and
controlling a display form of the displayed examination reservation information, based on the patient condition information.

12. A computer-readable storage medium on which is recorded a program for causing a computer to execute a process for assisting in a service rendered in a medical institution, the process comprising:
inputting preprocedure information which is composed of at least one of a type of a drug used for a preprocedure performed before an examination is conducted, an amount of the drug, an appliance used for the preprocedure, and a name of a person who performs the preprocedure;
inputting patient condition information, which is information indicating a condition of a patient after the preprocedure; and
correlating and storing the preprocedure information, the patient condition information, and a time of the preprocedure.

13. The storage medium according to claim 12, the process further comprising
presenting a correlation among the preprocedure information, the patient condition information, and the time of the preprocedure, which are stored.

14. The storage medium according to claim 12, the process further comprising:
displaying examination reservation information, which is reservation information of the examination; and
controlling a display form of the displayed examination reservation information, based on the patient condition information.

15. A computer data signal embodied in a carrier wave and representing a program, by being executed by a computer, for causing the computer to execute a process for assisting in a service rendered in a medical institution, the process comprising:
inputting preprocedure information which is composed of at least one of a type of a drug used for a preprocedure performed before an examination is conducted, an amount of the drug, an appliance used for the preprocedure, and a name of a person who performs the preprocedure;
inputting patient condition information, which is information indicating a condition of a patient after the preprocedure; and
correlating and storing the preprocedure information, the patient condition information, and a time of the preprocedure.

16. The computer data signal according to claim 15, the process further comprising
presenting a correlation among the preprocedure information, the patient condition information, and the time of the preprocedure, which are stored.

17. The computer data signal according to claim 15, further comprising:
displaying examination reservation information, which is reservation information of the examination; and
controlling a display form of the displayed examination reservation information, based on the patient condition information.

18. A medical service assisting system assisting in a service rendered in a medical institution, comprising:
preprocedure information inputting means for inputting preprocedure information which is composed of at least one of a type of a drug used for a preprocedure performed before an examination is conducted, an amount of the drug, an appliance used for the preprocedure, and a name of a person who performs the preprocedure;
condition inputting means for inputting patient condition information, which is information indicating a condition of a patient after the preprocedure; and
storing means for correlating and storing the preprocedure information, the patient condition information, and a time of the preprocedure.
